# EUROPEAN PATENT APPLICATION

(11) **EP 1 825 854 A1**
(43) Date of publication of application: **29.08.2007**
(21) Application number: 05816808.9
(22) Date of filing: 14.12.2005
(51) Int. Cl.: A61K 31/427, A61K 31/664, A61K 45/00, A61P 3/10, A61P 43/00

(54) **MEDICINAL COMPOSITION CONTAINING FBPase INHIBITOR**

(30) Priority: 15.12.2004 JP 2004362246
(71) Applicant: Daiichi Sankyo Company, Limited, Tokyo 103-8426 (JP)
(72) Inventor: YOSHIDA, Taishi, (JP); OKUNO, Akira, (JP)
(74) Representative: Gibson, Christian John Robert
(86) International application number: PCT/JP2005/022916
(87) International publication number: WO 2006/064826

(57) **Abstract**

To provide a method of treatment for diabetes mellitus having excellent decreasing activity in blood glucose levels and improving effects for dysfunction of β-cells in the pancreas of patients with diabetes mellitus.

A pharmaceutical composition comprising an FBPase inhibitor and an insulin sensitizer
5-[4-(6-methoxy-1-methyl-1H-benzimidazol-2-ylmethoxy)benzyl]thia zolidine-2,4-dione or a pharmacologically acceptable salt thereof.

## Description

### [Technical field]

The present invention relates to a pharmaceutical composition as a prophylactic or therapeutic agent for diabetes mellitus, hyperglycemia, impaired glucose tolerance, obesity, and diabetic complications (for example, retinopathy, cataract, nephropathy, peripheral nerve injury, and the like), steroid diabetes, glucose storage disease, diabetes like symptoms induced by surgery, glucose toxicity, functional failure of β-cells of the pancreas, and the like (preferably diabetes mellitus, diabetic complications, glucose toxicity, and functional failure of β-cells of the pancreas), comprising an FBPase inhibitor and an insulin sensitizer, which is 5-[4-(6-methoxy-1-methyl-1H-benzimidazol-2-ylmethoxy)benzyl]thia zolidine-2,4-dione or a pharmacologically acceptable salt thereof as active ingredients.

In addition, the present invention relates to the use of compounds described above for the manufacture of a pharmaceutical composition of the present invention described above, and a method for prophylaxis or treatment of the diseases described above by administration of the pharmaceutical composition described above to warm-blooded animals (preferably humans).

### [Background art]

Diabetes mellitus is a chronic metabolic disease and is characterized by hyperglycemia as a major symptom. It is said that the number of patients with diabetes mellitus is approximately 6.9 million in Japan, and approximately 150 million worldwide as of 1999, and is showing a tendency to increase annually. Thus it has been an important objective to develop a therapeutic procedure for diabetes mellitus and related disorders.

Chronically sustained hyperglycemia caused by diabetes mellitus results in dysfunction of the pancreas caused by decreased numbers of functional β-cells which synthesize and secrete insulin and thereby regulate blood glucose levels, by which in turn the prevalence of hyperglycemia and the severity of diabetes mellitus are furthermore induced (for example, see Non-patent Literature 1 and 2). Thus recently, from the viewpoint of prevention of severe diabetes mellitus, there is a particularly strong need to develop agents that can treat diabetes mellitus while protecting pancreatic β-cells.

It is known that FBPase inhibitors reduce blood glucose levels by suppression of gluconeogenesis due to inhibition of FBPase activity. In addition, it is known that insulin sensitizers lower blood glucose levels by improving dysfunction of insulin sensitivity and improve dysfunction of β-cells such as a decrease in insulin content and the like which are observed in diabetic status (for example, see Non-patent Literature 3 and 4) . However, the extent of improvement of β-cell function brought about by the insulin sensitizers so far is not sufficient for treating diabetes mellitus.

Furthermore, the lowering effects of combined administration of an insulin sensitizer and an FBPase inhibitor on blood glucose levels have been previously disclosed in Patent Literature 1, but hitherto it has not yet been known that combined administration of an insulin sensitizer and an FBPase inhibitor shows a remarkable improving effect on the function of β-cells in the patients with diabetes mellitus.
[Patent Literature 1] International publication number WO 00/52015 pamphlet
[Non-patent Literature 1] Endocrine Reviews (USA) Vol. 13, p. 415-31 (1992)
[Non-patent Literature 2] Diabetes (USA) Vol. 43, p. 1085-89 (1994)
[Non-patent Literature 3] Metabolism (USA) Vol. 53, No. 4, p. 488-494 (2004)
[Non-patent Literature 4] Diabetes (USA) Vol. 50, p. 1021-29 (2001)

### [Disclosure of the invention]

### [Object of the invention]

The present inventors have diligently conducted research to discover therapeutic procedures for diabetes mellitus, which exert excellent activities to reduce blood glucose levels and to improve dysfunction of pancreatic β-cells and found that the objective was achieved by combined administration of an insulin sensitizer 5-[4-(6-methoxy-1-methyl-1H-benzimidazol-2-ylmethoxy)benzyl]thia zolidine-2,4-dione or a pharmacologically acceptable salt thereof and an FBPase inhibitor, and consequently the inventors completed the present invention.

### [Means to achieve the object]

The present invention relates to
(1) a pharmaceutical composition comprising 5-[4-(6-methoxy-1-methyl-1H-benzimidazol-2-ylmethoxy)benzyl]thia zolidine-2,4-dione or a pharmacologically acceptable salt thereof and an FBPase inhibitor as active ingredients,
(2) a pharmaceutical composition according to (1) described above, wherein the FBPase inhibitor is an agent to inhibit human FBPase activity,
(3) a pharmaceutical composition according to (1) described above, wherein the FBPase inhibitor is a phosphoramide ester compound,
(4) a pharmaceutical composition according to (1) described above, wherein the FBPase inhibitor is 2-amino-5-isobutyl-4-{2-[5-(N,N'-bis((S)-1-ethoxycarbonyl)ethyl) phosphonamido]furanyl}thiazole or a pharmacologically acceptable salt thereof,
(5) a pharmaceutical composition according to any one selected from (1) to (4) described above, wherein 5-[4-(6-methoxy-1-methyl-1H-benzimidazol-2-ylmethoxy)benzyl]thia zolidine-2,4-dione or a pharmacologically acceptable salt thereof is 5-[4-(6-methoxy-1-methyl-1H-benzimidazol-2-ylmethoxy)benzyl]thia zolidine-2,4-dione hydrochloride,
(6) a prophylactic agent or a therapeutic agent for diabetes mellitus, which contains 5-[4-(6-methoxy-1-methyl-1H-benzimidazol-2-ylmethoxy)benzyl]thia zolidine-2,4-dione or a pharmacologically acceptable salt thereof and an FBPase inhibitor as active ingredients,
(7) a prophylactic agent or a therapeutic agent for diabetes mellitus, which is characterized by comprising 5-[4-(6-methoxy-1-methyl-1H-benzimidazol-2-ylmethoxy)benzyl]thia zolidine-2,4-dione or a pharmacologically acceptable salt thereof and an FBPase inhibitor as active ingredients and by improving function of β-cells in the pancreas,
(8) a prophylactic agent or a therapeutic agent for diabetes mellitus according to (6) or (7) described above, wherein the FBPase inhibitor is a phosphoramide ester compound,
(9) a prophylactic agent or a therapeutic agent for diabetes mellitus according to (7) or (8) described above, wherein the FBPase inhibitor is 2-amino-5-isobutyl-4-{2-[5-(N,N'-bis((S)-1-ethoxycarbonyl)ethyl) phosphonamido]furanyl}thiazole or a pharmacologically acceptable salt thereof,
(10) a prophylactic agent or a therapeutic agent for diabetes mellitus according to any one selected from (7) to (9) described above, wherein 5-[4-(6-methoxy-1-methyl-1H-benzimidazol-2-ylmethoxy)benzyl]thia zolidine-2,4-dione or a pharmacologically acceptable salt thereof is 5-[4-(6-methoxy-1-methyl-1H-benzimidazol-2-ylmethoxy)benzyl]thia zolidine-2,4-dione hydrochloride,
(11) a prophylactic agent or a therapeutic agent for diabetes mellitus according to any one selected from (6) to (10) described above, wherein said agent is an agent for a pharmaceutical composition,
(12) a prophylactic agent or a therapeutic agent for diabetes mellitus according to any one selected from (6) to (11) described above, wherein said pharmaceutical composition is packed for oral administration,
(13) an agent for improving the function of β-cells in the pancreas, which contains 5-[4-(6-methoxy-1-methyl-1H-benzimidazol-2-ylmethoxy)benzyl]thia zolidine-2,4-dione or a pharmacologically acceptable salt thereof and an FBPase inhibitor as active ingredients,
(14) an agent for improving the function of β-cells in the pancreas according to (13) described above, wherein the FBPase inhibitor is 2-amino-5-isobutyl-4-{2-[5-(N,N'-bis((S)-1-ethoxycarbonyl)ethyl) phosphonamido]furanyl}thiazole or a pharmacologically acceptable salt thereof,
(15) a prophylactic agent or a therapeutic agent for glucose toxicity, which contains 5-[4-(6-methoxy-1-methyl-1H-benzimidazol-2-ylmethoxy)benzyl]thia zolidine-2,4-dione or a pharmacologically acceptable salt thereof and an FBPase inhibitor as active ingredients,
(16) a prophylactic agent or a therapeutic agent for glucose toxicity according to (15) described above, wherein the FBPase inhibitor is 2-amino-5-isobutyl-4-{2-[5-(N,N'-bis((S)-1-ethoxycarbonyl)ethyl) phosphonamido]furanyl}thiazole or a pharmacologically acceptable salt thereof,
(17) an agent for reducing the glycosylated hemoglobin ratio, which contains 5-[4-(6-methoxy-1-methyl-1H-benzimidazol-2-ylmethoxy)benzyl]thia zolidine-2,4-dione or a pharmacologically acceptable salt thereof and an FBPase inhibitor as active ingredients,
(18) an agent for reducing the glycosylated hemoglobin ratio according to (17) described above, wherein the FBPase inhibitor is 2-amino-5-isobutyl-4-{2-[5-(N,N'-bis((S)-1-ethoxycarbonyl)ethyl) phosphonamido]furanyl}thiazole or a pharmacologically acceptable salt thereof as active ingredients,
(19) use of 5-[4-(6-methoxy-1-methyl-1H-benzimidazol-2-ylmethoxy)benzyl]thia zolidine-2,4-dione or a pharmacologically acceptable salt thereof and an FBPase inhibitor for the manufacture of a pharmaceutical composition comprising 5-[4-(6-methoxy-1-methyl-1H-benzimidazol-2-ylmethoxy)benzyl]thia zolidine-2,4-dione or a pharmacologically acceptable salt thereof and an FBPase inhibitor as active ingredients,
(20) use of 5-[4-(6-methoxy-1-methyl-1H-benzimidazol-2-ylmethoxy)benzyl]thia zolidine-2,4-dione or a pharmacologically acceptable salt thereof and an FBPase inhibitor for the manufacture of a pharmaceutical composition comprising 5-[4-(6-methoxy-1-methyl-1H-benzimidazol-2-ylmethoxy)benzyl]thia zolidine-2,4-dione or a pharmacologically acceptable salt thereof and an FBPase inhibitor for administration at the same time or independently at a certain time delay,
(21) use of 2-amino-5-isobutyl-4-{2-[5-(N,N'-bis((S)-1-ethoxycarbonyl)ethyl) phosphonamido]furanyl}thiazole or a pharmacologically acceptable salt thereof for the manufacture of a pharmaceutical composition according to (19) or (20) described above, wherein the FBPase inhibitor is 2-amino-5-isobutyl-4-{2-[5-(N,N'-bis((S)-1-ethoxycarbonyl)ethyl) phosphonamido]furanyl}thiazole or a pharmacologically acceptable salt thereof, and
(22) a therapeutic procedure for diabetes mellitus, which is
characterized by treating diabetes mellitus while improving the function of β-cells in the pancreas by combined administration of 5-[4-(6-methoxy-1-methyl-1H-benzimidazol-2-ylmethoxy)benzyl]thia zolidine-2,4-dione or a pharmacologically acceptable salt thereof and an FBPase inhibitor.

In the present invention, an "FBPase inhibitor" is not particularly restricted provided that it inhibits FBPase (fructose-1,6-bisphosphatase) activity, and it is, for example, a phosphoramide ester compound containing the phosphoramide ester structure as a prodrug moiety, which is disclosed in International publication number WO 01/47935 pamphlet, or a compound disclosed in International publication number WO 00/14095 pamphlet, Journal of Medicinal Chemistry, Vol. 45, 3865-3877, 2002, and Bioorganic & Medicinal Chemistry Letters, Vol. 11, 17-21, 2001, preferably a phosphoramide ester compound, and particularly preferably 2-amino-5-isobutyl-4-{2-[5-(N,N'-bis((S)-1-ethoxycarbonyl)ethyl) phosphonamido]furanyl}thiazole or a pharmacologically acceptable salt thereof.

An "insulin sensitizer" in the present invention is a general name for an agent that improves insulin resistance and enhances insulin sensitivity.

5-[4-(6-Methoxy-1-methyl-1H-benzimidazol-2-ylmethoxy)benzyl ]thiazolidine-2,4-dione or a pharmacologically acceptable salt thereof is preferably
5-[4-(6-methoxy-1-methyl-1H-benzimidazol-2-ylmethoxy)benzyl]thia zolidine-2,4-dione hydrochloride.
5-[4-(6-Methoxy-1-methyl-1H-benzimidazol-2-ylmethoxy)benzyl]thia zolidine-2,4-dione or a pharmacologically acceptable salt thereof can be manufactured according to methods disclosed in Japanese Patent Publication (Kokai) Number Hei 9-295970, EP 0745600, USP 5,886,014, and International publication number WO 00/71540 pamphlet.

A pharmaceutical composition of the present invention is efficacious for prophylaxis or therapy of, for example, diabetes mellitus, hyperglycemia, impaired glucose tolerance, obesity, diabetic complications (for example, retinopathy, cataract, nephropathy, peripheral nerve injury, and the like), steroid diabetes, glucose storage disease, diabetes-like symptoms induced by surgery, glucose toxicity, functional failure of β-cells of the pancreas, and the like, and preferably for prophylaxis or treatment of diabetes mellitus, diabetic complications, glucose toxicity, and functional failure of β-cells of the pancreas.

In the present invention, one or more FBPase inhibitors can be used.

An FBPase inhibitor and 5-[4-(6-methoxy-1-methyl-1H-benzimidazol-2-ylmethoxy)benzyl]thia zolidine-2,4-dione or a pharmacologically acceptable salt thereof can be administered as a pharmaceutical composition. In addition, each active ingredient can be individually and simultaneously administered. Furthermore, the active ingredients can be separately administered one after another with an appropriate time interval. The acceptable time intervals for achieving beneficial effects brought about by administration of these agents can be determined and confirmed in clinical treatments or animal studies.

The pharmaceutical composition of the present invention can be administered in various dosage forms. The dosage forms are not particularly restricted and can be determined depending on a various factors such as the formulation and the age, sex and other characteristics of patients as well as the severity of the disease, and the like. For example, in the case of tablets, pills, powder, granules, syrup, liquids and solutions, suspensions, emulsions, granules, and capsules, they are orally administered.

These pharmaceutical preparations are prepared according to conventional methods using additive agents known in the formulation field of remedies such as excipients, binders, disintegrants, lubricants, dissolution agents, flavors, coating agents, and the like.

When tablets are prepared, inactive ingredients conventionally employed in this field may be widely used. For instance, they are excipients such as lactose, sucrose, sodium chloride, glucose, urea, starch, calcium carbonate, kaolin, crystalline cellulose, silicic acid, and the like; binders such as water, ethanol, propanol, syrup, glucose solution, starch solution, gelatin solution, carboxymethylcellulose, shellac, methylcellulose, potassium phosphate, polyvinylpyrrolidone, and the like; disintegrants such as dried starch, sodium alginate, agar powder, laminaran powder, sodium hydrogen carbonate, calcium carbonate, polyoxyethylene sorbitan fatty acid esters, sodium lauryl sulfate, stearic acid monoglyceride, starch, lactose, and the like; disintegration inhibitors such as sucrose, stearin, cacao butter, hydrogenated oil; absorption enhancers such as quaternary ammonium bases, sodium lauryl sulfate, and the like; humectants such as glycerin, starch, and the like; adsorbents such as starch, lactose, kaolin, bentonite, colloidal silicic acid, and the like; lubricants such as purified talc, stearates, boric acid powder, polyethylene glycol, and the like. In addition, tablets may be conventionally coated when required, and, for example, they may be prepared as sugar-coated tablets, gelatin-coated tablets, enteric-coated tablets, film-coated tablets, or double-coated tablets, and multi-layer tablets.

When pills are prepared, well known inactive ingredients that are conventionally employed in this field may be widely used. For instance, excipients such as glucose, lactose, starch, cacao butter, hardening vegetable oil, kaolin, talc, and the like; binders such as gum arabic acacia powder, tragacanth powder, gelatin, ethanol, and the like; disintegrants such as laminaran, agar, and the like may be exemplified.

Furthermore, coloring agents, preservatives, perfumes, flavors, sweetening agents, and other remedies may be contained, when required.

The amount of the active ingredients contained in the above preparations is not particularly restricted and may be selected from a wide range, but it is usually from 1 to 70 weight percent of the total weight of the preparation, and preferably, the appropriate amount is from 1 to 30 weight percent.

The dose of each agent and the dose-ratio of the two active ingredients for diabetes mellitus employed in the present invention may vary significantly depending on a variety of factors such as the activity of each agent, the symptoms, age, and body weight of the patients, and the like.

The amount of FBPase inhibitor contained in the above preparations is not particularly restricted and may be selected from a wide range, but it is usually from 1 to 70 weight percent of the total weight of the preparation, and preferably, the appropriate amount is from 1 to 40 weight percent.

The dose varies depending on a variety of factors such as the symptoms, age, and body weight of the patients, the formulation, and the like. A suitable dosage level is generally from 0.001 mg (preferably 0.01 mg, and more preferably 0.1 mg) per day as a lower limit to 2000 mg (preferably 200 mg, and more preferably 100 mg) per day as an upper limit for human adult.

The amount of 5-[4-(6-methoxy-1-methyl-1H-benzimidazol-2-ylmethoxy)benzyl]thia zolidine-2,4-dione or a pharmacologically acceptable salt thereof contained in the above preparations is not particularly restricted and may be selected from a wide range, but it is usually from 1 to 70 weight percent of the total weight of the preparation, and preferably, the appropriate amount is from 1 to 30 weight percent.

The dose varies depending on a variety of factors such as the symptoms, age, and body weight of the patient, the formulation, and the like. A suitable dosage level is generally from 0.001 mg (preferably 0.01 mg, and more preferably 0.1 mg) per day as a lower limit to 2000 mg (preferably 200 mg, and more preferably 20 mg) per day as an upper limit for human adult.

In the present invention, an FBPase inhibitor and 5-[4-(6-methoxy-1-methyl-1H-benzimidazol-2-ylmethoxy)benzyl]thia zolidine-2,4-dione or a pharmacologically acceptable salt thereof can be administered at a dose specified for each active ingredient described above simultaneously or separately at an appropriate time interval, either once a day or at several times throughout the day with the dosage being divided into several sub-doses.

### [Advantages of the invention]

According to the present invention, blood glucose levels are remarkably decreased and dysfunction of β-cells in the pancreas is also significantly improved by combined administration of an insulin sensitizer, 5-[4-(6-methoxy-1-methyl-1H-benzimidazol-2-ylmethoxy)benzyl]thia zolidine-2,4-dione or a pharmacologically acceptable salt thereof, and an FBPase inhibitor, and consequently, diabetes mellitus can be effectively prevented or improved. In addition, said pharmaceutical composition is also effective against diabetic complications caused by hyperglycemia. Furthermore, the pharmaceutical composition of the present invention is expected to exert prophylactic and therapeutic effects by inducing rapid improvement of hyperglycemia and stable decreasing effects on blood glucose levels with extremely few adverse events even in a long-term administration by appropriate selection of agents, dosing methods, doses, and the like depending on the symptoms. From these advantages, the pharmaceutical composition of the present invention is considered to provide a prophylactic agent and a therapeutic agent for various diseases described hereinbefore.

### [Best mode for carrying out the invention]

The present invention is illustrated in more detail by the following test examples. However, the present invention is not limited to these examples.

### [Examples]

Inhibitory actions of test compounds against FBPase activity can be determined according to methods disclosed in International publication number WO 00/14095 pamphlet.
2-Amino-5-isobutyl-4-{2-[5-(N,N'-bis((S)-1-ethoxycarbonyl)ethyl) phosphonamido]furanyl}thiazole (Compound A) can be synthesized according to methods disclosed in International publication number WO 01/47935 pamphlet.

The insulin sensitizer, 5-[4-(6-methoxy-1-methyl-1H-benzimidazol-2-ylmethoxy)benzyl]thia zolidine-2,4-dione hydrochloride (Compound B), can be synthesized according to methods described in Japanese Patent Publication (Kokai) Number Hei 9-295970, EP 0745600, USP 5,886,014, and International publication number WO 00/71540 pamphlet.

The insulin sensitizer, 5-(4-((6-hydroxy-2, 5, 7, 8-tetramethylchroman-2-yl)methoxy)benzyl) -2,4-thiazolidinedione (Troglitazone; Compound T), can be synthesized according to methods described in USP 4,572,912, and Japanese Patent Publication (Kokai) Number Hei 2-31079.

### [Test Example 1]

Effects of co-administration of an FBPase inhibitor, 2-amino-5-isobutyl-4-{2-[5-(N,N'-bis((S)-1-ethoxycarbonyl)ethyl) phosphonamido]furanyl}thiazole or a pharmacologically acceptable salt thereof (Compound A), and
5-[4-(6-methoxy-1-methyl-1H-benzimidazol-2-ylmethoxy)benzyl]thia zolidine-2,4-dione hydrochloride (Compound B)

Eight week old male Zucker diabetic fatty (ZDF) rats that spontaneously developed diabetes mellitus (purchased from Charles River Japan Inc.) were divided into 6 groups as follows: control group, Compound A administered group (Group A), Compound B administered group (Group B), Compound T administered group (Group T), Compound B and Compound A co-administered group (Group B+A), and Compound T and Compound A co-administered group (Group T+A) . Rats in the control group were allowed to take powdered chow (powdered FR-2, Funabashi Farm Co., Ltd.) *ad libitum* for 6 weeks.

Powdered chow containing Compound A at a concentration of 0.2 % for Group A, Group B+A and Group T+A, that containing Compound B at a concentration of 0.4 ppm for Group B and Group B+A, and that containing an insulin sensitizer, troglitazone (Compound T), at a concentration of 0.1% for Group T and Group T+A were prepared, and rats were allowed to take the powdered chow containing one of the test compounds or two of the test compounds prepared for each group, respectively, *ad libitum* for the same period. No decreases in food intake were detected in any group throughout the observation period.

6 weeks after administration of the test compounds was started, blood sample was collected from the tail vein of each rat, and ratios of glycosylated hemoglobin (HbA1c) were determined by DCA2000 (Bayer Medical Ltd.). Since glycosylated hemoglobin is non-enzymatically formed by binding of glucose to hemoglobin and the ratio of glycosylated hemoglobin is increased by sustained hyperglycemia, the glycosylated hemoglobin ratio is widely used as an indicator of a long-term therapy (or control of blood glucose levels) in patients with diabetes mellitus (N. Eng J. Med., vol. 310, p. 341-346 (1984)).

After administration of the test compounds was terminated, the rats were sacrificed and the pancreas was isolated for determination of the pancreatic insulin content. Insulin in the pancreas was extracted by a hydrochloric acid-ethanol extraction method (Diabetelogia, vol. 27, p. 454-459 (1984)), and the concentration of insulin in the extracts was determined by rat insulin RIA kit (Linco Research Inc.) to calculate the pancreatic insulin content. Pancreatic insulin content is widely used as an indicator of β-cell status in the pancreas, that is an insulin-secreting cell, since this level is known to decrease along with the progression of diabetes mellitus (Proc. Natl. Acad. Sci. USA, vol. 96, p. 10857-10862 (1999), Diabetes, vol. 48, p. 2398-2406 (1999), and the like).

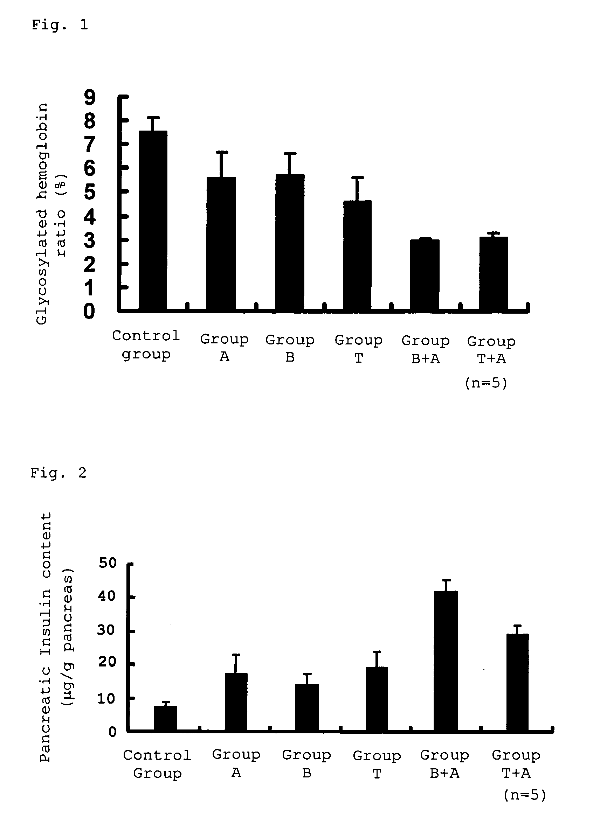

The average and standard error of the glycosylated hemoglobin ratio and the pancreatic insulin content in each group following administration of the compound(s) for 6 weeks are shown in Fig. 1 and Fig. 2, respectively.

Figure 1 shows that the glycosylated hemoglobin ratio in the blood was remarkably reduced in both Group B+A (Compound A and Compound B co-administered group) and Group T+A (Compound T and Compound A co-administered group) without weakening the individual effects of each compound (by two way analysis of variance). From these findings, it was clearly demonstrated that combined administration of an insulin sensitizer and an FBPase inhibitor is useful as a method of treatment for patients with diabetes mellitus.

Figure 2 shows that the pancreatic insulin content was synergistically increased following co-administration of an FBPase inhibitor (Compound A) and Compound B (by two way analysis of variance). On the other hand, although co-administration of Compound A and Compound T increased insulin levels in the pancreas without weakening the effects of each compound, the increase was not synergistic, but only additive (by two way analysis of variance).

Thus it was demonstrated that although the decrease in the glycosylated hemoglobin ratio in the co-administration group of Compound A and Compound B (Group B+A) was roughly the same as that in the co-administration group of Compound A and Compound T (Group T+A) (Fig. 1, p=0.4108, t-test), the pancreatic insulin content in the co-administration of Compound A and Compound B (Group B+A) was synergistically increased. Therefore it is considered that combined administration of an insulin sensitizer (Compound B) plus an FBPase inhibitor is a very useful therapeutic procedure for patients with diabetes mellitus, which exerts an improving action on the function of β-cells in the pancreas.

Although the reasons why dysfunction of β-cells in the pancreas such as a decrease in insulin content and the like is caused in diabetic status are not fully understood, chronic hyperglycemia itself in diabetic status is considered to be one of the reasons (Endocrine Review (USA), vol. 13, p. 415-31 (1992)). When hyperglycemia is sustained for a long term, glycosylation of various proteins is induced in various tissues and organs, and additionally, oxidative stress is increased in many tissues due to activation of the electron transfer system in mitochondria. β-Cells in the pancreas are not exception. Indeed, since the expression of enzymes related to antioxidant metabolism is low in the pancreas (0-15% of that in the liver), the pancreas is more susceptible to oxidative stress than other tissues and organs. As a result, the function of β-cells in the pancreas is considered to be exacerbated. This concept is referred to as "glucose toxicity". The results of the present Test Example 1 that the glycosylated hemoglobin ratio in the blood was improved and additionally the pancreatic β-cells insulin content was increased by co-administration of an insulin sensitizer and an FBPase inhibitor are considered to be mainly brought about by relieving glucose toxicity in the pancreas.

Generally, an agent referred to as an insulin sensitizer is a ligand to a nuclear receptor, PPARγ, and is considered to improve hyperglycemia by activation or inactivation of transcription of various proteins involved not only in glucose metabolism and lipid metabolism, but also in differentiation and proliferation of adipocytes. However, it remains unknown which protein transcription is important to be activated or inactivated for the improvement of hyperglycemia. Furthermore, although Actos (Takeda Pharmaceutical Industries) and Avandia (GlaxoSmithKline GmbH & Co KG) are currently available insulin sensitizers, results of their clinical studies exhibited different actions on lipid metabolism and the like (Drug, vol. 63, p. 1373-405 (2003)).

Considering these results and informations, the reasons why co-administration of an FBPase inhibitor and Compound B, an insulin sensitizer, improved function of β-cells in the pancreas more potently than co-administration of an FBPase inhibitor and Compound T, another insulin sensitizer, even though hyperglycemia was improved by roughly the same level, are probably due to differences in potency of improving activity towards the function of β-cells between these two insulin sensitizers.

From these considerations, a combined administration of an insulin sensitizer
5-[4-(6-methoxy-1-methyl-1H-benzimidazol-2-ylmethoxy)benzyl]thia zolidine-2,4-dione or a pharmacologically acceptable salt thereof, and an FBPase inhibitor is considered a promising novel therapeutic procedure for patients with diabetes mellitus, because it is possible to control blood glucose at favorable levels while improving the function of β-cells in the pancreas.

### [Brief description of the drawings]

### Figure 1.

This figure shows changes in the glycosylated hemoglobin ratio in the blood following co-administration of an FBPase inhibitor (Compound A) and an insulin sensitizer (Compound B or Compound T) for 6 weeks (Test Example 1).

### Figure 2.

This figure shows changes in the pancreatic insulin content following co-administration of an FBPase inhibitor (Compound A) and an insulin sensitizer (Compound B or Compound T) for 6 weeks (Test Example 1).

## Claims

1. A pharmaceutical composition, comprising 5-[4-(6-methoxy-1-methyl-1H-benzimidazol-2-ylmethoxy)benzyl]thia zolidine-2,4-dione or a pharmacologically acceptable salt thereof and an FBPase inhibitor as active ingredients.

2. A pharmaceutical composition according to Claim 1, wherein the FBPase inhibitor is an agent to inhibit human FBPase activity.

3. A pharmaceutical composition according to Claim 1, wherein the FBPase inhibitor is a phosphoramide ester compound.

4. A pharmaceutical composition according to Claim 1, wherein the FBPase inhibitor is 2-amino-5-isobutyl-4-{2-[5-(N,N'-bis((S)-1-ethoxycarbonyl)ethyl) phosphonamido]furanyl}thiazole or a pharmacologically acceptable salt thereof.

5. A pharmaceutical composition according to any one claim from Claim 1 to Claim 4, wherein 5-[4-(6-methoxy-1-methyl-1H-benzimidazol-2-ylmethoxy)benzyl]thia zolidine-2,4-dione or a pharmacologically acceptable salt thereof is 5-[4-(6-methoxy-1-methyl-1H-benzimidazol-2-ylmethoxy)benzyl]thia zolidine-2,4-dione hydrochloride.

6. A prophylactic agent or a therapeutic agent for diabetes mellitus, which contains 5-[4-(6-methoxy-1-methyl-1H-benzimidazol-2-ylmethoxy)benzyl]thia zolidine-2,4-dione or a pharmacologically acceptable salt thereof and an FBPase inhibitor as active ingredients.

7. A prophylactic agent or a therapeutic agent for diabetes mellitus, which is **characterized by** comprising 5-[4-(6-methoxy-1-methyl-1H-benzimidazol-2-ylmethoxy)benzyl]thia zolidine-2,4-dione or a pharmacologically acceptable salt thereof and an FBPase inhibitor as active ingredients and by improving the function of β-cells in the pancreas.

8. A prophylactic agent or a therapeutic agent for diabetes mellitus according to Claim 6 or Claim 7, wherein the FBPase inhibitor is a phosphoramide ester compound.

9. A prophylactic agent or a therapeutic agent for diabetes mellitus according to Claim 7 or Claim 8, wherein the FBPase inhibitor is 2-amino-5-isobutyl-4-{2-[5-(N,N'-bis((S)-1-ethoxycarbonyl)ethyl) phosphonamido]furanyl}thiazole or a pharmacologically acceptable salt thereof.

10. A prophylactic agent or a therapeutic agent for diabetes mellitus according to any one claim selected from Claim 7 to Claim 9, wherein
5-[4-(6-methoxy-1-methyl-1H-benzimidazol-2-ylmethoxy)benzyl]thia zolidine-2,4-dione or a pharmacologically acceptable salt thereof is
5-[4-(6-methoxy-1-methyl-1H-benzimidazol-2-ylmethoxy)benzyl]thia zolidine-2,4-dione hydrochloride.

11. A prophylactic agent or a therapeutic agent for diabetes mellitus according to any one claim selected from Claim 6 to Claim 10, wherein the agents are comprised in a pharmaceutical composition.

12. A prophylactic agent or a therapeutic agent for diabetes mellitus according to any one claim selected from Claim 6 to Claim 11, which is a pharmaceutical composition for oral administration.

13. An agent for improving the function of β-cells in the pancreas, which contains 5-[4-(6-methoxy-1-methyl-1H-benzimidazol-2-ylmethoxy)benzyl]thia zolidine-2,4-dione or a pharmacologically acceptable salt thereof and an FBPase inhibitor as active ingredients.

14. An agent for improving the function of β-cells in the pancreas according to Claim 13, wherein the FBPase inhibitor is
2-amino-5-isobutyl-4-{2-[5-(N,N'-bis((S)-1-ethoxycarbonyl)ethyl) phosphonamido]furanyl}thiazole or a pharmacologically acceptable salt thereof.

15. A prophylactic agent or a therapeutic agent for glucose toxicity, which contains
5-[4-(6-methoxy-1-methyl-1H-benzimidazol-2-ylmethoxy)benzyl]thia zolidine-2,4-dione or a pharmacologically acceptable salt thereof and an FBPase inhibitor as active ingredients.

16. A prophylactic agent or a therapeutic agent for glucose toxicity according to Claim 15, wherein the FBPase inhibitor is
2-amino-5-isobutyl-4-{2-[5-(N,N'-bis((S)-1-ethoxycarbonyl)ethyl) phosphonamido]furanyl}thiazole or a pharmacologically acceptable salt thereof.

17. An agent for reducing the glycosylated hemoglobin ratio, which contains
5-[4-(6-methoxy-1-methyl-1H-benzimidazol-2-ylmethoxy)benzyl]thia zolidine-2,4-dione or a pharmacologically acceptable salt thereof and an FBPase inhibitor as active ingredients.

18. An agent for reducing the glycosylated hemoglobin ratio according to Claim 17, wherein the FBPase inhibitor is
2-amino-5-isobutyl-4-{2-[5-(N,N'-bis((S)-1-ethoxycarbonyl)ethyl) phosphonamido]furanyl}thiazole or a pharmacologically acceptable salt thereof.

19. Use of
5-[4-(6-methoxy-1-methyl-1H-benzimidazol-2-ylmethoxy)benzyl]thia zolidine-2,4-dione or a pharmacologically acceptable salt thereof and an FBPase inhibitor for the manufacture of a pharmaceutical composition comprising
5-[4-(6-methoxy-1-methyl-1H-benzimidazol-2-ylmethoxy)benzyl]thia zolidine-2,4-dione or a pharmacologically acceptable salt thereof and an FBPase inhibitor as active ingredients.

20. Use of
5-[4-(6-methoxy-1-methyl-1H-benzimidazol-2-ylmethoxy)benzyl]thia zolidine-2,4-dione or a pharmacologically acceptable salt thereof and an FBPase inhibitor for the manufacture of a pharmaceutical composition comprising
5-[4-(6-methoxy-1-methyl-1H-benzimidazol-2-ylmethoxy)benzyl]thia zolidine-2,4-dione or a pharmacologically acceptable salt thereof and an FBPase inhibitor for administration at the same time or independently at a certain time delay.

21. Use of
2-amino-5-isobutyl-4-{2-[5-(N,N'-bis((S)-1-ethoxycarbonyl)ethyl) phosphonamido]furanyl}thiazole or a pharmacologically acceptable salt thereof for the manufacture of a pharmaceutical composition according to Claim 19 or Claim 20, wherein the FBPase inhibitor is 2-amino-5-isobutyl-4-{2-[5-(N,N'-bis((S)-1-ethoxycarbonyl)ethyl) phosphonamido]furanyl}thiazole or a pharmacologically acceptable salt thereof.

22. A therapeutic procedure of diabetes mellitus, which is **characterized by** treating diabetes mellitus while improving the function of β-cells in the pancreas by combined administration of 5-[4-(6-methoxy-1-methyl-1H-benzimidazol-2-ylmethoxy)benzyl]thia zolidine-2,4-dione or a pharmacologically acceptable salt thereof and an FBPase inhibitor.
